# EUROPEAN PATENT APPLICATION

(11) **EP 1 522 308 A1**
(43) Date of publication of application: **13.04.2005**
(21) Application number: 03078182.7
(22) Date of filing: 08.10.2003
(51) Int. Cl.: A61K 35/78, A23L 1/30, A23L 1/304, A61P 1/04, A61P 35/00

(54) **Use of chlorophyll or analogues for the prevention of cytotoxic effects of dietary heme on the intestinal mucosa**

(71) Applicant: Stichting Top-Instituut Voedselwetenschappen, 6700 AN Wageningen (NL)
(72) Inventor: De Vogel, Johan, NL-6709 RM Wageningen (NL); Jonker-Tremont, Denise, NL-6661 KK Elst (NL); Van der Meer, Roelof, NL-6712 HA Ede (NL)
(74) Representative: van Westenbrugge, Andries

(57) **Abstract**

Diets high in red meat are associated with increased risk for colon cancer which is at least in part caused by the heme content of red meat. The cytotoxicity of heme causes an increased exposure of colonic epithelial cells to luminal irritants and results in colonic epithelial hyperproliferation, hence increasing the risk of cells acquiring mutations and subsequent development of malignancies. The present invention relates to methods wherein chlorophyll or analogues thereof are applied for the prevention of cytotoxic effects of heme on the intestinal mucosa. The invention aims at the use of chlorophyll or analogues thereof to specifically counteract the effects of dietary heme. The protective activity of chlorophyll can be enhanced by the addition of calcium. Therefore, according to the present invention, chlorophyll or analogues thereof may be applied in foodstuffs containing red meat and other sources of dietary free heme, to protect the colonic mucosa against heme induced toxicity and to prevent the onset and progression of colonic malignancies.

## Description

### Field of the invention

The present invention relates to methods wherein chlorophyll or analogues thereof are applied for the prevention of cytotoxic effects of heme on the intestinal mucosa. The invention aims at the use of chlorophyll or analogues thereof to specifically detoxify dietary heme. Therefore, according to the present invention, chlorophyll or analogues thereof may be applied in foodstuffs containing red meat and other sources of dietary heme, to protect the colonic mucosa against heme induced toxicity and to prevent the onset and progression of colonic malignancies.

### Background of the invention

In Western societies, colon cancer is one of the major causes of cancer death. For the United States in 2002, nearly 110,000 new cases were reported and almost 48,000 patients die annually because of this disease, making colorectal cancer the second leading cause of cancer death in the US [1].

There is a wide geographic variation in colon cancer incidence; the rates of incidence vary 20-fold between high- and low-risk countries [2]. Migrant and other epidemiological studies indicate that this variation is due to environmental factors, with diet as a major determinant [3]. Diets high in red- and processed meat are especially associated with a moderate but increased risk for colon cancer [4]. Remarkably, diets high in white meat (poultry, fish) are not associated with an increased risk [5, 6]. Although it is recognized that genetic factors are important determinants for the genesis of colorectal cancer in individuals [7], it appears that differences in colon cancer incidence are largely attributable to environmental factors [8]. Epidemiological studies have shown that especially people with a Western-style diet (high meat, high fat, low fiber) are at high risk for colon cancer. Incidence increases in countries with a high meat consumption [3].

Previously studies have been performed to explain the association of red meat and colorectal malignancies. Several hypothesis have been tested, such as heterocyclic aromatic amines, nitrosating agents like NO and N₂O₃ which can form N-nitroso compounds, an increased intake of dietary fat which is accompanied by an increase in membrane-damaging bile acids and lastly an increase in dietary iron. A clear role in the increase of colorectal malignancies could not be established for any of these compounds.

For these reasons the Sesink et al. [9], hypothesized that heme, the iron-protoporphyrin pigment of red meat might be an important dietary risk factor, instead of earlier proposed meat-associated mutagens such as heterocyclic amines or N-nitroso compounds [10]. Sesink et al. showed in rat studies that dietary heme causes an increase in the cytotoxicity of the fecal water, indicating an increased exposure of the colonic epithelial cells to luminal irritants, resulting in colonic epithelial hyperproliferation [9]. As mentioned above, this hyperproliferation increases the risk of endogenous mutations in cell-turnover genes and is therefore considered as an early risk marker for colon cancer [11, 12].

Heme is a porphyrin that is coordinated with Fe(II). The ubiquitous heme molecule serves as functional part of a wide variety of proteins, such as hemoglobin, myoglobin, nitric oxide synthase and cyclo-oxygenase, and is therefore crucial for various cellular processes. Myoglobin, hemoglobin, transferrin and ferritin are abundant in red meat and are prime sources of heme uptake in most western diets.

Heme is quite hydrophobic and readily enters cell membranes, which greatly increases cellular susceptibility to oxidant-mediated killing. Heme also acts as a catalyst for the oxidation of low-density lipoprotein (LDL), generating products toxic to endothelium. Although heme can be directly cytotoxic, investigations established that hemoglobin-derived heme and iron might also be indirectly toxic through the generation of oxidized forms of low-density lipoprotein (LDL).

Iron and iron containing complexes such as heme encourage production and propagation of free radicals which can damage DNA and presumably increase cancer risk. In a study of over 14,000 individuals, high iron intake and high iron body stores were both positively linked to the risk of colon cancer. Higher levels of iron were associated with higher incidence of colon polyps, possible forerunners of colon tumors [13].

Based on mutational analysis of colon cancers, Kinzler and Vogelstein, [14] argued that dietary factors that lead to colon cancer are probably not mutagens, but rather luminal irritants that damage colonic epithelial cells. This triggers a compensatory epithelial hyperproliferation, which increases the risk of endogenous mutations in tumor suppressor and oncogenes. Clonal accumulation of these endogenous mutations may eventually result in the adenoma-carcinoma sequence of colon carcinogenesis [15, 16]. Heme is one factor that triggers hyperproliferation of the colonic epithelium and thereby increases the risk of the onset and propagation of colonic malignancies and cancer.

Effects of dietary phytochemicals on cancer development and particularly chemoprevention of carcinogenesis by phytochemicals have been studied extensively. A large amount of epidemiological studies indicate that vegetables protect against coloncancer, especially green and raw vegetables [17, 18]. Dietary vegetables, fruits, and whole grains contain a wide variety of phytochemicals that have the potential to modulate cancer development. There are many biologically plausible reasons why consumption of plant foods might slow or prevent the appearance of cancer. These include the presence in plant foods of such potentially anticarcinogenic substances as carotenoids, chlorophyll, flavonoids, indole, isothiocyanate, polyphenolic compounds, protease inhibitors, sulfides, and terpens. The specific mechanisms of action of most phytochemicals in cancer prevention are not yet clear but appear to be varied. Considering the large number and variety of dietary phytochemicals, their interactive effects on cancer risk may be extremely difficult to assess. Phytochemicals can inhibit carcinogenesis by inhibition and induction of enzymes, scavenging of DNA reactive agents such as free radicals and reactive oxygen species (ROS), suppression of the abnormal proliferation of early, preneoplastic lesions, and inhibition of certain properties of the cancer cell.

Naturally occurring chlorophylls (Chl) have shown anti-mutagenic activity. The chlorophyll mediated reduction of the formation *in vivo* of DNA adducts by potent environmental carcinogens have been studied in animal models such as rainbow trout [19]. Chlorophyll was further shown to inhibit 1,2 dimethylhydrazine-induced nuclear damage in rat colonic epithelium [20].

Chlorophyll is safe for human consumption and currently employed for colouring fats, oils, soaps, and alcoholic beverages, and it has been suggested as a food additive in nutraceuticals, as an anti-oxidant, deodorant and anti-inflammatory compound.

The art however does not disclose that green vegetables offer protection against heme toxicity. In particular the art does not disclose that high chlorophyll concentration in green vegetables and fruits specifically, or chlorophyll alone, may be used specifically to prevent heme induced cytotoxicity and hyperproliferation of colonic epithelial cells. It is thus an object of the current invention to provide for compositions, foodstuffs and diets containing purposely elevated levels of chlorophyll or analogues thereof for use in methods for the prevention of the heme-induced hyperproliferative effects associated with heme-containing diets, e.g. diets rich in red meat, that will ultimately lead to a reduced risk of developing colonic malignancies and cancer.

### Detailed description of the invention

The present invention is based on the surprising discovery that chlorophyll and analogues thereof can counter the cytotoxic effects of heme on the intestinal mucosa. The cytotoxicity of dietary heme induces hyperproliferation of colonic epithelial cells [9] and promotes formation of aberrant crypt foci in rat colon [21]. This hyperproliferation of the colonic epithelium increases the endogenous mutation rate and thereby increases the risk of developing colon cancer [11, 14]. The discovery that chlorophyll can counter these adverse effects of heme on the intestinal epithelium thus allows the use of chlorophyll containing compositions for human consumption for the prevention of cytotoxic effects of heme-containing diets thereby ultimately preventing, or at least reducing the risk of developing colon cancer.

In a first aspect the invention therefore relates to a method for reducing or preventing the adverse effects of heme on the intestinal mucosa. More in particular the invention relates to a method for reducing or preventing the adverse effects of heme on the intestinal epithelium. These adverse effects of heme include the cytotoxicity of heme, i.e. the cytotoxic effect of dietary heme on the intestinal mucosa and/or epithelium. Preferably the method is a method for preventing or reducing the cytotoxicity and hyperproliferation cytotoxic effect of dietary heme on the intestinal mucosa and/or epithelium. More particularly, the method is a method for preventing or reducing the cytotoxicity and hyperproliferative effects of dietary heme on the colonic mucosa and/or epithelium. The method of the invention thereby ultimately is a method for the prevention of, or at least to offset an increased risk of developing an intestinal neoplasm, of which in particular colon-cancer.

The method of the invention comprises the administration of a source of chlorophyll. The source of chlorophyll is preferably administered so as to achieve an effective concentration of chlorophyll or an analogue thereof in the intestinal tract. The administration of the source of chlorophyll is therefore preferably by oral administration, more preferably, the source of chlorophyll is consumed. The source of chlorophyll may be administered or consumed as such, i.e. as a pharmaceutical or neutraceutical composition, or as part of a diet or a meal, i.e. as a food supplement or food ingredient.

Preferably in the method of the invention, the source of chlorophyll is administered to a subject that is consuming a heme-containing meal or that is on a heme-containing diet, more preferably the subject is consuming a meal rich in heme or that is on a diet rich in heme. Examples of a diet or meal rich in heme include diets or meal that include or are rich in red meat. A diet rich in heme is herein understood to be a diet in which the average daily intake of heme is at least 0.05, 0.1, 0.2 or 0.5 mmol heme per kg dry weight of diet.

Preferably in the method of the invention, the source of chlorophyll is administered during a time frame that overlaps with the period of the cytotoxic activity of the heme consumed in the diet or meal. The source of chlorophyll may thus be administered/consumed sequential to the consumption of heme, as well as simultaneous with the consumption of heme. In case of sequential administration, the source of chlorophyll is preferably administered before the consumption of heme.

The source of chlorophyll for use in the method of the invention may be any composition comprising chlorophyll or a chlorophyll analogue. Chlorophyll analogues include chlorophyllin, bacteriochlorophyll and Fe²⁺, Mg²⁺, Cu²⁺ or Zn²⁺ containing chlorophyll's and/or analogues thereof. The source of chlorophyll may be obtained from a variety of well-known, preferably natural sources. Higher plants and green algae, such as Chlorella contain chlorophyll a and chlorophyll b in the approximate ratio of 3:1. The molecular formula of chlorophyll a is C₅₅H₇₂MgN₄O₅; the molecular formula of chlorophyll b is C₅₅H₇₀MgN₄O₆. The difference between the two chlorophylls is that a methyl side-chain in chlorophyll a is replaced by a formyl group in chlorophyll b. Chlorophyll a is found with chlorophyll c in many types of marine algae. Red algae contain principally chlorophyll a and also chlorophyll d.

Chlorophyll sources include e.g. natural and transgenic chlorophyll containing plants, vegetables, fruits, sprouts, green algae and seaweeds. In particular vegetables containing high levels of chlorophyll may be advantageously used, such as the following non-limiting list of: spinach, kale, parsley, broccoli, green cabbage, wheatgrass, barleygrass, alfalfa and lettuce. Green algea such as Chlorella sp. , Spirulina sp., Aphanizomenon flosaquae and others, may used to produce extracts or dried powders that are particularly rich in chlorophyll. Chlorophyll containing cyanobacteria and preparations derived therefrom may also be used. Genetically modified plants, algae and/or bacteria that have been modified to produce elevated levels of chlorophyll may be advantageously used as sources of chlorophyll.

In the method of the invention, the source of chlorophyll is preferably administered/consumed in an amount effective to reduce or prevent the adverse effects of heme on the intestinal mucosa as described above. The amount of the source of chlorophyll that is administered or consumed can be varied. Typically the amount of the source of chlorophyll provides for chlorophyll or analogues thereof in an amount that is at least 0.1, 0.2, 0.5, 1.0, 2.0, 5,0 or 10 times the average amount of heme in the diet, on a molar basis. Preferably, the source of chlorophyll that is administered/consumed comprises at least 0.02, 0.1, 0.2, 0.5, 1.0, 2.0 mmol of chlorophyll or analogue thereof per kg dry weight diet. Typically, 0.01, 0.05, 0.1, 0.2 0.5, 1.0, 2.0 mmol of chlorophyll or an analogue thereof is administered or consumed on a daily basis.

In a preferred embodiment of the method, the source of chlorophyll is administered or consumed in the form of a pharmaceutical or neutraceutical composition, e.g. together with a pharmaceutical carrier. The preferred formulation of such pharmaceutical or neutraceutical compositions depends on the intended mode of administration and therapeutic application. The pharmaceutical carrier can be any compatible, non-toxic substance suitable to deliver the source of chlorophyll to the patient. Pharmaceutically acceptable carriers are well known in the art and include, for example, aqueous solutions such as (sterile) water or physiologically buffered saline or other solvents or vehicles such as glycols, glycerol, oils such as olive oil or (injectable) organic esters, alcohol, fats, waxes, and inert solids may be used as the carrier. A pharmaceutically acceptable carrier may further contain physiologically acceptable compounds that act, e.g. to stabilise or to increase the absorption of the source of chlorophyll. Such physiologically acceptable compounds include, for example, carbohydrates, such as glucose, sucrose or dextrans, antioxidants, such as ascorbic acid or glutathione, chelating agents, low molecular weight proteins or other stabilisers or excipients. One skilled in the art would know that the choice of a pharmaceutically acceptable carrier, including a physiologically acceptable compound, depends, for example, on the route of administration of the composition. Pharmaceutically acceptable adjuvants, buffering agents, dispersing agents, and the like, may also be incorporated into the pharmaceutical compositions.

For oral administration in the methods of the present invention, the source of chlorophyll can be administered in solid dosage forms, such as capsules, tablets, and powders, or in liquid dosage forms, such as elixirs, syrups, solutions and suspensions. The source of chlorophyll can be encapsulated in gelatin capsules together with inactive ingredients and powdered carriers, such as glucose, lactose, sucrose, mannitol, starch, cellulose or cellulose derivatives, magnesium stearate, stearic acid, sodium saccharin, talcum, magnesium carbonate and the like. Examples of additional inactive ingredients that may be added to provide desirable color, taste, stability, buffering capacity, dispersion or other known desirable features are red iron oxide, silica gel, sodium lauryl sulphate, titanium dioxide, edible white ink and the like. Similar diluents can be used to make compressed tablets. Both tablets and capsules can be manufactured as sustained release products to provide for continuous release of the chlorophyll or analogue thereof over a period of hours. Compressed tablets can be sugar coated or film coated to mask any unpleasant taste and protect the tablet from the atmosphere, or enteric-coated for selective disintegration in the gastrointestinal tract. Liquid dosage forms for oral administration can contain colouring and flavouring to increase patient acceptance.

In another preferred embodiment of the method, the source of chlorophyll is administered or consumed in the form of a food product or foodstuff, whereby the source of chlorophyll may e.g. be a food constituent, ingredient or additive. The food product can be any substance that is suitable for consumption by humans and can be either a ready-to-eat food product or a constituent that can be incorporated in or processed to give a food product. The food can be a solid, semi-solid or fluid food, can be a fermented or non-fermented food and can be a food that is served and/or consumed hot or cold. The term food as used herein thus includes any kind of drinks or beverages.

Preferably the food product comprises edible plant material comprising a source of chlorophyll. Preferably the edible plant material is a plant material that is rich in chlorophyll such as e.g., parsley, broccoli, green cabbage, alfalfa and other sprouts, various kinds of lettuce and the group of vegetables known as leafy greens, which includes the leaves from spinach, kale, Swiss chard beet greens, collards, mustard greens, turnip greens, arugula greens, sorrel greens, dandelion greens and bok choy.

A preferred food product for use in the methods of the present invention may be enriched with a source of chlorophyll. A food product enriched with a source of chlorophyll may thus comprise, preferably in addition to edible plant materials comprising chlorophyll, added chlorophyll obtained from sources as described above, including e.g. natural and transgenic chlorophyll containing plants, vegetables, fruits, sprouts, green algae and seaweeds. The added chlorophyll may be isolated or purified from these sources by methods known in the art.

In a preferred embodiment of the invention, the food product further comprises one or more ingredients comprising heme, such e.g. meat (steaks, cold cuts, hams, minced meat, sausages, meat-sauces, offal), of which in particular red meat. In such instances, the source of chlorophyll is thus administered or consumed together or simultaneously with the heme, or at least as part of one meal. A large variety of heme-containing food products may thus be supplemented with a source of chlorophyll in order to reduce or prevent the adverse effect of heme-consumption. Such food-products include e.g. ready-to-eat food products, including dough products like noodles, pasta, soups and the like; processed meat products such as sausage, canned and also products such as instant meals meals, diet foods, complete foods and baby food, sauces, desserts, snacks. Particularly preferred food products in heme-containing food ingredients are combined with a source of chlorophyll include instant meals. Various forms of instant meals are available in the art including e.g. frozen meals, fresh meals that are refrigerated for a limited period of time (e.g. less than two weeks) or sterilized or desiccated meals that may be stored at room temperature for a prolonged period of time (e.g. more than 1 month). Instant meals may be precooked, requiring only to be thawed and heated in e.g. a microwave. Alternatively, instant meals may be partially prepared, requiring to be cooked, steamed or fried before consumption.

The amount of the source of chlorophyll in the food product can be varied, but typically the amount of the source of chlorophyll provides for chlorophyll or analogues thereof in an amount that is at least 0.1, 0.2, 0.5, 1.0, 2.0, 5,0 or 10 times the average amount of heme in the food product (on a molar basis) for an optimal reduction of the heme induced adverse effects.

In a further preferred embodiment of the invention, the use of a source of chlorophyll in accordance to the invention for the prevention and/or reduction of adverse effects of dietary heme, is combined with calcium as second protective compound against the negative effects of heme consumption, further enhancing the protective effect of chlorophyll. The combined use of chlorophyll and calcium as chemoprotectants against heme toxicity is particularly preferred. In the methods of the invention, the administration or consumption of a source of chlorophyll may thus advantageously be combined with the administration or consumption of a source of calcium.

In the methods of the invention preferably a source of chlorophyll and a source of calcium are co-administered (or co-consumed). Here by co-administration (or co-consumption) is meant that on the one hand the source of chlorophyll and on the other the source of calcium are administered during a time frame wherein the respective periods of (pharmacological) activity overlap. Thus the term includes sequential as well as simultaneous administration. Since the source of chlorophyll and the source of calcium may be co-administered in a sequential as well as in a coextensive fashion the term "formulation" as used in this context in this document should be understood to encompass pharmaceutical/neutraceutical kits which comprise separate dosage units for the source of chlorophyll component and the source of calcium. Thus, the formulation may comprise a composition comprising both the source of chlorophyll and the source of calcium, allowing for simultaneous administration of both components. Alternatively, the formulation may be a kit comprising separate dosage units for the source of chlorophyll and for the source of calcium, allowing for the sequential addition of both components. Likewise the term "formulation" in the context of the present invention relates to food products or even meals in which the source of chlorophyll and the source of calcium may be consumed in a sequential as well as in a coextensive fashion. Thus, e.g. one food product in a meal may comprise the source of chlorophyll and another food product may comprise a source of calcium, or both sources may be consumed in a single food product.

The source of calcium in the formulation or food product of the invention comprises a composition capable of releasing Ca²⁺ ions in the gastrointestinal tract upon administration or consumption. Preferably, the source of calcium is suitable for human consumption, i.e. is physiologically acceptable, which will usually mean that it is not toxic or otherwise harmful to human or animals when administered orally or consumed. Preferably, the source of calcium is a calcium salt, preferably a calcium salt soluble in the stomach. Examples of preferred calcium salts include e.g. calcium carbonates, calcium acetate, calcium lactate, calcium chloride, calcium phosphates, of which calcium lactate is most preferred.

The source of calcium is consumed, administered and/or are present in the formulation of the invention in an amount effective in reducing or preventing the adverse effects of heme as defined above. For these purposes usually at least 1000, 2000, 3000, 4000, 5000 mg calcium per kg dry weight of food, foodstuff or drink is administered. A single dose of calcium may be administered every day or every other day. The pharmaceutical and/or neutraceutical compositions and formulations comprising a source of calcium for use in the method of the invention can be solid, semi-solid or fluid dosage forms as described above, and may thus also comprise a source of chlorophyll.

For countries outside the US, the above defined sources of chlorophyll, preferably in combination with a source of calcium, may be used for the manufacture of any of the above defined compositions (pharmaceutical, neutraceutical and food products) for the prevention and/or reduction of any of the adverse effects of heme as defined above in accordance with any of the methods as defined above.

The use of chlorophyll containing compositions for this particular purpose, and advertizing the health-promoting effects of the added chlorophyll in combination with heme-containing food or diets, on chlorophyll enriched food products and neutraceuticals or diets is also an aspect of the current invention.

### Examples

### Materials and methods

### Animals and diets

The experimental protocols were approved by the animal welfare committee of Wageningen University. About eight-week-old, outbred male Wistar rats (Harlan Horst/WU, SPF) were housed individually in metabolic cages in a room with controlled temperature (± 20°C), relative humidity (50-60%) and a light/dark cycle (6 AM to 6 PM). Animals were acclimatized to housing conditions for 5 days before start of the experiment.

In the first study we tested whether spinach prevents the effect of dietary heme. Mean body weight of the animals was 324 ± 3 g. During two weeks, four groups of eight rats were fed purified diets. The control group consumed a diet which contained per kg, 200 g casein, 532 g dextrose, 200 g fat (160 g palm fat and 40 g corn oil), 20 g cellulose and 20 mmol calcium phosphate (CaPO₄·2H₂O, Fluka Chemie Buchs, Switzerland). The heme and heme plus spinach diets were supplemented with 0.5 mmol heme / kg diet (hemin was used for this purpose, Sigma-Aldrich Chemie, St Louis, MI, US). The spinach diets contained per kg diet 82 g powdered freeze-dried spinach at the expense of cellulose and dextrose, with a chlorophyll concentration of approximately 1,2 mmol chlorophyll / kg diet. We measured the chlorophyll concentration of spinach (Iglo, 's-Hertogenbosch) after a five-time repeated extraction with acetone / water (80:20, v/v) comparing the absorption spectra with standard chlorophyll solutions (Sigma) on a spectrophotometer (Perkin Elmer, Lambda 2, Norwalk, MO, US). Dietary concentrations of cellulose, calcium, potassium, protein, fat and carbohydrate content were similar for all diets. Vitamins and other minerals were added according to the recommendations of the American institute of Nutrition 1993 [22].

In the second study, using a similar experimental design as the spinach study, we tested whether the effects of spinach are due to the chlorophyll content. The body weight of the animals at the start of this study was 275 ± 1 g (mean ± SE). The new diet was supplemented with heme and chlorophyll. This diet was prepared as a heme diet (as described above), with the addition of 12 g chlorophyll solution in palm oil (1,2 mmol chlorophyll / kg diet, E-140, Japan Chlorophyll CO, Chiyoda-ku, Tokyo) instead of 12 g palm fat. The control, heme and heme plus spinach diets were also prepared as described above. The only difference was 12 g palm oil (Japan Chlorophyll CO, Chiyoda-ku, Tokyo) instead of 12 g palm fat / kg diet as a placebo for the chlorophyll solution.

Food and demineralised drinking water were supplied ad libitum. Food intake and body weights were recorded every 2-4 days. Feces were collected quantitatively during days 11-14 of the experiment and were frozen immediately at -20°C.

### In vivo colonic epithelial cell proliferation.

After the experimental feeding period of 14 days, we quantified the colon epithelial cell proliferation as DNA-replication, using [methyl-³H]thymidine incorporation into DNA. We choose this biochemical method because it is the most rapid, specific and quantitative marker of cell replication *in vivo.* Moreover, we have shown earlier that DNA-replication highly correlates with faecal water cytotoxicity [23]. This substantiates a cause-and-effect relationship that we want to address in the present study. Non-fasted rats were injected i.p. with [methyl-³H]thymidine (specific activity 925 GBq / mmol; dose 3.7 MBq / kg body weight; Amersham International, Amersham, UK) in 154 mM NaCl. After 2 hours the rats were killed by CO₂ inhalation. The colon was removed and opened longitudinally. Colonic contents were removed by rinsing with 154 mM KCl. The mucosa was scraped using a spatula and homogenized (Ultraturrax Pro200: Pro Scientific Inc., Monroe, CT, US) in 1 ml buffer containing 200 mM sucrose, 20 mM tris, 1 mM dithiothreitol at pH 7.4, with a protease inhibitor cocktail (Roche Diagnostics GmbH, Mannheim, Germany). These homogenates were analyzed for ³H-thymidine incorporation and for total DNA content. The macromolecular fraction was precipitated in 5% trichloroacetic acid (wt/vol), followed by 10 min centrifugation at 10.000 g. The supernatant was discarded and the pellet resuspended in 1 M perchloric acid and hydrolyzed twice at 70°C for 10 min. A sample (0.5 ml) of the hydrolysate was dissolved in 15 ml Aqua Luma (Lumac-LSC BV., Groningen, The Netherlands) and counted with corrections for quenching on a Beckman LS-7500 liquid scintillation counter. DNA content was determined using the diphenylamine reaction with calf thymus DNA as standard (Sigma) [24].

### Quantification of epithelial DNA in feces.

Fecal host DNA content was quantified using DNA extraction and real-time PCR, using rat-specific probe and primers for the β-globin gene. In short, DNA was isolated from 20 mg freeze-dried feces using the QIAamp DNA stool mini kit (Westburg, Leusden, The Netherlands) following the recommendations of the manufacturer for human DNA analysis. All isolates were of good purity (A260/A280 ∼ 1.8) and were stored at 4°C or at -20°C for long-term storage. The standard DNA used for the quantification of the DNA in the feces was isolated from rat spleen, using the Wizard Genomic DNA purification kit (Promega Corporation, Madison, WI, USA). The real-time PCR was performed using the ABI prism 7700 Sequence Detection system (PE Applied Biosystems, Nieuwerkerk a/d IJssel, The Netherlands). Universal Taqman Master Mix and Taqman Exogenous Internal positive Control (IPC) Reagent (Vic ™ probe) were purchased from Applied Biosystems and the Taqman probe containing a 5' FAM labeled fluorescent reporter dye (6-carboxyfluorosein) and a 3' TAMRA quencher dye (6-carboxytetramethylrhodamine) was purchased from Proligo (Paris, France). PCR primers were synthesized by Amersham Pharmacia Biotech Custom DNA Synthesis Service (Roosendaal, The Netherlands). For amplification of host DNA 5 µl of the purified DNA (100-500 ng) was used in a volume of 50 µl, containing 1x Master Mix, 200 nM forward primer (5'-TGATGGCCTGAAACACTTGG-3'), 200 nM reverse primer (5'-TCAGGATCCACATGCAGCTT-3'), 100 nM probe (5'-CAACCTCAAGGGCACCTTTGCTCA-3'), 1x IPC mix and 1x IPC DNA. The PCR protocol consisted of 2 min 50°C, 10 min 95°C and 40 cycles of 15 sec 95°C and 1 min 62°C.

### Preparation of fecal water.

Fecal water was prepared by reconstituting a small amount of freeze-dried feces with double-distilled water to 20 % dry weight. After homogenizing, samples were incubated for 1 hour in a shaking water bath at 37°C, with mixing every 15 min, followed by centrifugation for 20 min at 13.500 g. The supernatants were collected and centrifuged again at 14.000 g for 2 min. The osmolarity (Osmomat 030-D, Gonotec, Berlin, Germany) of the fecal waters was determined. If osmolarity differed from 300 mOsmol/L, another portion of freeze-dried feces was reconstituted with double-distilled water, adjusting the percentage dry weight to obtain fecal water with an osmolarity of 300 mOsmol/L. The fecal waters were stored at -20°C until further analysis.

### Cytotoxicity of fecal water

Cytotoxicity of fecal water was quantified by potassium release after incubation with a human erythrocyte suspension as described previously [9] and validated earlier with intestinal epithelial cells [25]. The potassium content of the erythrocytes was measured with an Inductive Coupled Plasma Absorption Emission Spectrophotometer (ICP-AES, Varian, Mulgrave, Australia) and the cytotoxicity of fecal water was calculated and expressed as a percentage of maximal lysis.

### Purification of the cytotoxic heme factor from fecal water.

Pooled fecal water from the different treatment groups was first purified, by adding 560 µl water and 2.5 ml diethylether (ether) to 100 µl fecal water, and after vigorous mixing, centrifugation at 1500 g for 5 minutes. The ether phase containing chlorophyll was discarded and the procedure was repeated 2 times. The remaining 660 µl sample was acidified with 60 µl HCl (final HCl concentration 1 M) and further extracted with chloroform / methanol as described earlier [26]. The lipid-containing chloroform phase was obtained after centrifugation (10 min, 1500 g) and evaporated under nitrogen. These extracts were purified by size exclusion chromatography (SEC) as follows: A Waters 501 Pumping System (Waters Corporation, Milford, MA, US) was used, in combination with a photo diode array detector (Shimadzu, Japan) to determine the absorption spectrum of the eluted compounds and a fraction collector (Ultrorac II, LKB, Brommen, Sweden). We used the absorption at 400 nm for detection of porphyrin structures. Dried lipid extracts of 100 µl fecal water were solubilized in 250 µl of the elution solvent. Then, 200 µl were injected via a Gilson 231 autosampler (Gilson Medical Electronics Inc., Middleton, US) fitted with a 500 µl loop. The sample was isocratically separated on a Jordi GPC / divinylbenzene column (300 x 7.8 mm, 5 µm, 500Å; Jordi Associates, Bellingham, MA, US). Chloroform / methanol / acetonitrile / triethylamine (55:30:10:5, v/v/v/v) was used as the solvent with a flow rate of 1.0 ml / min. The data was processed and analyzed using Shimadzu software. Fractions were collected in one minute intervals between 0 and 12 min after injection and evaporated under a nitrogen-stream. The cytotoxicity of these fractions was determined as described above, after resolubilising the samples in 50 mM NaOH and adjusting to neutral pH and 300 mOsm/L with 3-[N-morpholino]-propanesulfonic acid (MOPS).

### Statistical Analysis.

All results are expressed as means ± SEM (n=8 per group). A commercially available package (Statistica 6.1, Statsoft Inc., Tulsa, USA) was used for all statistics. In case of normally distributed data (Shapiro Wilk test), analysis of variance (ANOVA) was tested between groups and homogeneity of variances was tested using the Levene's test. Significant differences between means of different dietary groups were tested with a two-sided Student's *t* test (equal variance) or the Student's *t* test with Welch's estimate (unequal variance). When data were not normally distributed, Kruskall-Wallis ANOVA was used and in case of significant treatment effects, the non-parametric Mann-Whitney U test was used as a post-hoc test. Differences were considered statistically significant when P < 0.05.

### Example 1

### Spinach inhibits the colonic effects of heme.

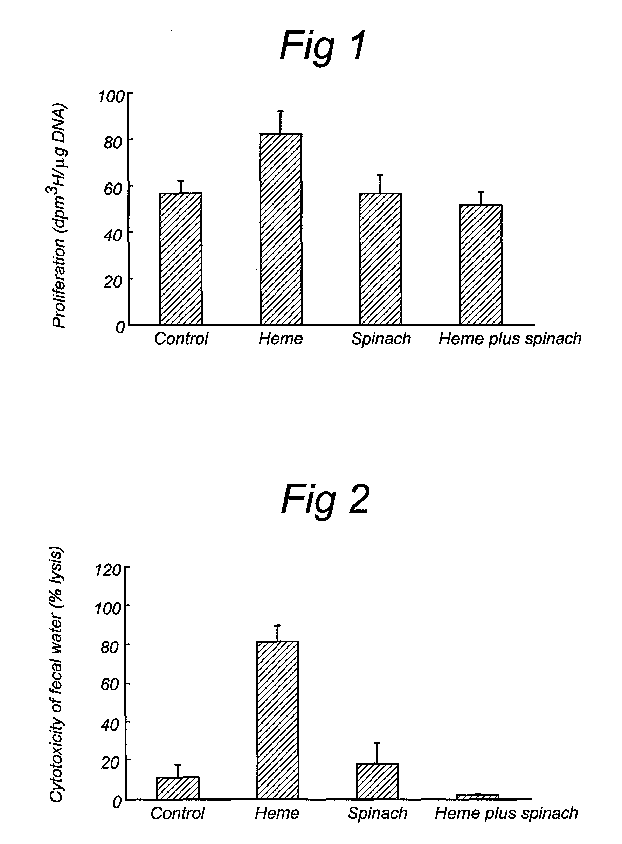

Addition of spinach to the diets did not result in a different food intake (17.0 g / day ± 1.2 g, mean ± SEM) or growth rate (2.0 g / day ± 0.6 g) between the groups. To study the response of the colonic epithelium to the different diets, the proliferation of the colonic epithelial cells was measured. The dietary heme group had a 50 % increase in rate of DNA-replication compared with the control group. Supplementation of spinach to the heme diet resulted in a total inhibition of this heme-induced colonic epithelial hyperproliferation. Moreover, the rats fed the spinach diet without heme showed no difference in epithelial proliferation compared with the control group (Figure 1). Heme treatment resulted even in a decreased DNA content of the mucosa by 20 % compared with the other groups (4207 µg ± 150 µg DNA / g scraping and 5297 µg DNA ± 309 µg / g scraping, respectively). Therefore, these differences in proliferation reflect differences in cell turnover.

Subsequently, we determined whether these differences in cell turnover were due to differences in exposure of the colonocytes to luminal irritants. The cytotoxicity of the heme group was increased 7-fold compared with the control group. The addition of spinach to the heme diet abolished any cytotoxic effects of heme. Furthermore, the supplementation of the diet with spinach alone did not affect cytotoxicity compared with the fecal water of the control group (Figure 2).

These data show that spinach inhibits the heme-induced cytotoxic and hyperproliferative effects in the colon.

### Example 2

### The protective effect of spinach is due to its chlorophyll content

To study the effect of chlorophyll, we used a study design similar to the spinach experiment. We replaced the dietary spinach group for a heme plus chlorophyll group, with a concentration of chlorophyll equimolar to the heme plus spinach group. Food intakes of the different groups were approximately 18 g / day. The growth rate of the rats fed heme plus chlorophyll was slightly higher than that of the other rats (5.4 ± 0.3 g / day and 4.2 ± 0.3 g / day respectively).

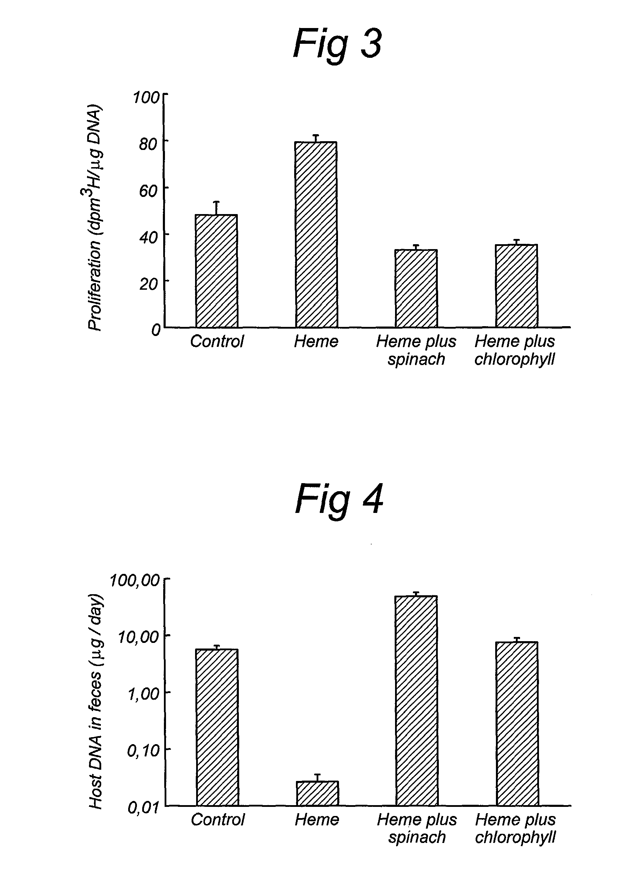

The effect of the different diets on the proliferation of the colon epithelial cells was very reproducible: generally the same effects were observed for the control, heme and heme plus spinach diets in the chlorophyll study as in the spinach study. Figure 3 shows that chlorophyll and spinach both completely prevented the heme-induced epithelial hyperproliferation.

Subsequently, we determined whether these diet-induced differences in cell turnover resulted in different exfoliation of colonocytes into the fecal stream. Figure 4 shows that heme in the diet of the rats caused a strong reduction of host DNA detected in the feces, compared to the control diet. Spinach and chlorophyll supplemented to the heme diet totally abolished the heme-induced effects.

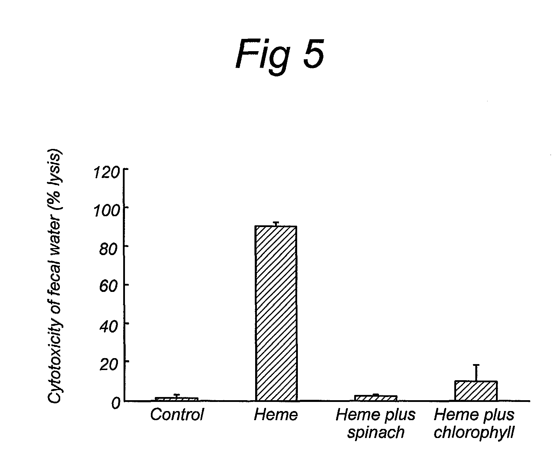

Furthermore, supplementation of heme resulted again in an increase of the cytotoxicity of the fecal water compared with the control diet, similar to the effect of heme observed in the spinach study. Supplementation of chlorophyll and spinach to the heme diet inhibited this heme-induced increase completely (Figure 5).

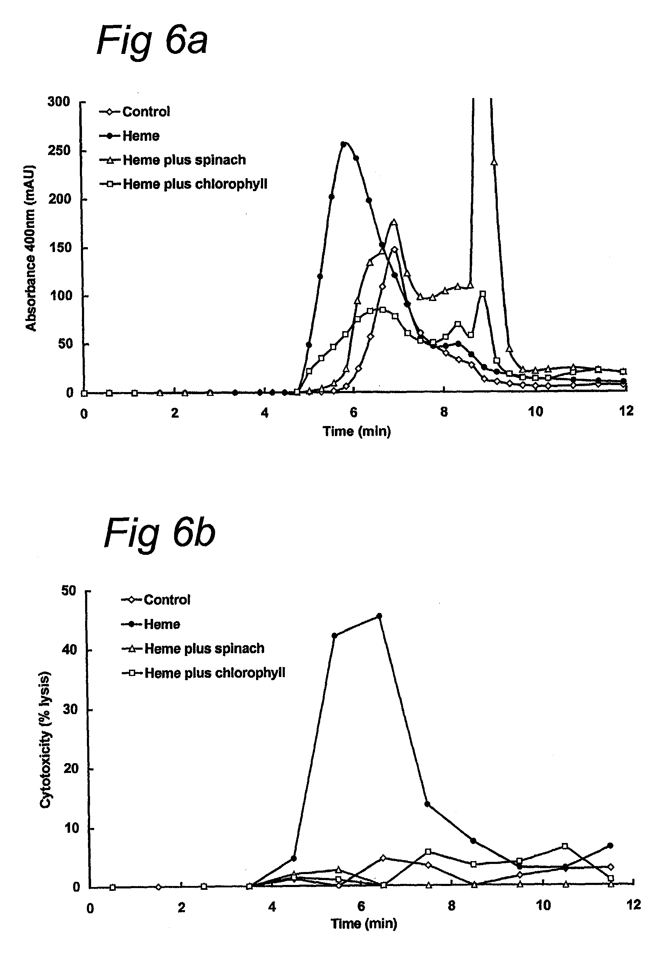

Finally, we tried to purify the cytotoxic factor responsible for the heme effects described above. First we determined whether the cytotoxic components in the fecal water were lipid soluble, given the lipid soluble character of known other cytotoxic agents such as bile acids. The cytotoxicity of these lipid extracts was the same or even higher then that of the corresponding fecal waters (data not shown), showing that the cytotoxic factor is completely extracted in the chloroform phase. To further purify the cytotoxic heme factor, we subjected the different lipid extracts to size exclusion chromatography (SEC). This technique separates high from low molecular weight components, using a hydrophobic solvent. We measured the absorption at 400 nm to quantify porphyrin structures. Figure 6A shows the chromatograms of the lipid extracts of the chlorophyll study. The heme fecal water contained high-molecular weight components eluting around 6 min. It should be noted that a heme standard added to this fecal water eluted at about 12 min (data not shown). The control, heme plus spinach and heme plus chlorophyll groups contained lower-molecular weight components eluting around 7 and 9 min. The identity of the components in the control, heme plus spinach, and heme plus chlorophyll groups eluting around 7 min is still unclear. However, the absorption spectra of the components eluting around 9 min showed similarities with the spectrum of chlorophyll and were therefore designated as chlorophyll metabolites. Subsequently, we determined the cytotoxicity of all the collected fractions of the different groups at time intervals indicated in Figure 6B. The cytotoxicity of the high-molecular weight components eluting around 6 min of the heme group was very high. However, no significant cytotoxic activity was found in any of the fractions of the other dietary groups. Furthermore, in a separate experiment, all the material from a heme faecal water extract eluting from the column was collected and its cytotoxicity corresponded with the cytotoxicity of the original lipid extract, showing that none of the cytotoxic components were trapped on the column (data not shown).

These examples 1 and 2 show for the first time that spinach or chlorophyll inhibit the heme-induced stimulation of colonic epithelial cell-turnover. Our present results of the rat DNA quantification from faeces show that heme inhibited this exfoliation of colonocytes almost completely. The heme-induced increase in rate of cell division and decrease in exfoliation of the epithelial cells indicate that heme alters the life cycle of colonocytes by inducing death in pre-senescent cells. Remarkably, these heme-induced effects on colonocyte turnover were prevented by supplementation of the heme diet with spinach. Moreover, this protective effect of spinach can be mimicked completely by an equivalent amount of chlorophyll, indicating that other components, such as other phytochemicals fibres in spinach are not involved.

The molecular structural similarity of chlorophyll and heme will prevent heme from interacting with various cellular components by competition with chlorophyll for binding to and/or reacting with these cellular components. Moreover, chlorophyll is known to form sandwich-like structures and will interact at the molecular level with heme to form sandwich-like structures.

### Figure legends

Fig. 1. Effect of dietary heme, spinach and heme plus spinach on the colonic epithelial proliferation, determined by [methyl-³H]thymidine incorporation in colon mucosa of rats (mean ± SEM, n=8). An asterisk means significantly different from control group (P < 0.05).
Fig. 2. Effect of dietary heme, spinach and heme plus spinach on the cytotoxicity of fecal water of rats (means ± SEM, n=8). Cytotoxicity was determined using an erythrocyte bioassay. An asterisk means significantly different from control group (P < 0.05).
Fig. 3. Effect of dietary heme, heme plus spinach and heme plus chlorophyll on the colonic epithelial proliferation, determined by [methyl-³H]thymidine incorporation in colon mucosa of rats (mean ± SEM, n=8). An asterisk means significantly different from control group (P < 0.05).
Fig. 4. Effect of dietary heme, heme plus spinach and heme plus chlorophyll on the level of host DNA detected in feces of rats. Host DNA was quantified by real-time PCR as described in Materials and Methods, using rat-specific β-globin probe - primer combinations (means ± SEM, n=8). An asterisk means significantly different from control group (P < 0.05).
Fig. 5. Effect of dietary heme, heme plus spinach and heme plus chlorophyll on the cytotoxicity of fecal water of rats (means ± SEM, n=8). Cytotoxicity was determined using an erythrocyte bioassay. An asterisk means significantly different from control group (P < 0.05).
Fig. 6. Representative size-exclusion chromatograms of lipid extracts of pooled fecal waters from rats fed a control, heme, heme plus spinach or a heme plus chlorophyll diet, measured by the absorbance at 400 nm (A). One minute fractions were collected from 0 to 12 min and assayed for cytotoxicity (B).

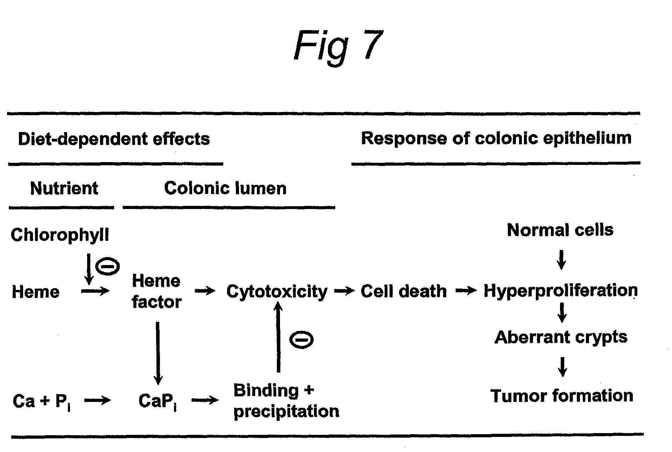

Fig. 7. Proposed mechanism for the interaction between dietary heme, calcium phosphate and chlorophyll in the lumen of the colon and the effects on the colonic epithelium.

1. American Cancer Society, *Cancer Facts & Figures.* 2002, New York: American Cancer Society Inc., *http:*//*www. cancer. org*/*downloads*/*STT*/*CancerFacts&Figures2002TM.pdf*
2. Potter, J.D., *Colorectal cancer: molecules and populations*. J Natl Cancer Inst, 1999. **91**(11): p. 916-32.
3. Armstrong, B. and R. Doll, *Environmental factors and cancer incidence and mortality in different countries, with special reference to dietary practices.* Int J Cancer, 1975. **15**(4): p. 617-31.
4. Norat, T., et al., *Meat consumption and colorectal cancer risk: dose-response meta- analysis of epidemiological studies.* Int J Cancer, 2002. **98**(2): p. 241-56.
5. Giovannucci, E., et al., *Intake of fat, meat, and fiber in relation to risk of colon cancer in men.* Cancer Res, 1994. **54**(9): p. 2390-7.
6. Willett, W.C., et al., *Relation of meat, fat, and fiber intake to the risk of colon cancer in a prospective study among women.* N Engl J Med, 1990. **323**(24): p. 1664-72.
7. Fearon, E.R. and P.A. Jones, *Progressing toward a molecular description of colorectal cancer development.* Faseb J, 1992. **6**(10): p. 2783-90.
8. MacLennan, R., *Diet and colorectal cancer.* Int J Cancer, 1997. **Suppl 10:** p. 10-2.
9. Sesink, A.L.A., et al., *Red meat and colon cancer: the cytotoxic and hyperproliferative effects of dietary heme.* Cancer Res, 1999. **59**(22): p. 5704-9.
10. Bingham, S.A., *Epidemiology and mechanisms relating diet to risk of colorectal cancer*, in *Nutrition Research Reviews.* 1996: Cambridge. p. 197-239.
11. Lipkin, M., *Biomarkers of increased susceptibility to gastrointestinal cancer: new application to studies of cancer prevention in human subjects.* Cancer Res, 1988. **48**(2): p. 235-45.
12. Baron, J.A., *Intermediate effect markers for colorectal cancer.* IARC Sci Publ, 2001. **154**: p. 113-29.
13. Nelson, R.L., et al., *Body iron stores and risk of colonic neoplasia.* J Natl Cancer Inst, 1994. **86**(6): p. 455-60.
14. Kinzler, K.W. and B. Vogelstein, *Lessons from hereditary colorectal cancer.* Cell, 1996. **87**(2): p. 159-70.
15. Terdiman, J.P., *Genomic events in the adenoma to carcinoma sequence.* Semin Gastrointest Dis, 2000. **11**(4): p. 194-206.
16. Fearon, E.R. and B. Vogelstein, *A genetic model for colorectal tumorigenesis.* Cell, 1990. **61**(5): p. 759-67.
17. Trock, B., E. Lanza, and P. Greenwald, *Dietary fiber, vegetables, and colon cancer: critical review and meta- analyses of the epidemiologic evidence.* J Natl Cancer Inst, 1990. **82**(8): p. 650-61.
18. Steinmetz, K.A. and J.D. Potter, *Vegetables, fruit, and cancer prevention: a review.* J Am Diet Assoc, 1996. **96**(10): p. 1027-39.
19. Harttig, U. and G.S. Bailey, *Chemoprotection by natural chlorophylls in vivo: inhibition of dibenzo[a,l]pyrene-DNA adducts in rainbow trout liver.* Carcinogenesis, 1998. **19**(7): p. 1323-6.
20. Robins, E.W. and R.L. Nelson, *Inhibition of 1,2-dimethylhydrazine-induced nuclear damage in rat colonic epithelium by chlorophyllin.* Anticancer Res, 1989. **9**(4): p. 981-5.
21. Pierre, F., Taché, S., Petit, C. R., Van Der Meer, R., Corpet, D. E., *Meat and cancer: haemoglobin and haemin in a low calcium diet promote colorectal* *carcinogenesis at the aberrant crypt stages in rats.* Carcinogenesis, 2003. **In press.**
22. Reeves, P.G., F.H. Nielsen, and G.C. Fahey, Jr., *AIN-93 purified diets for laboratory rodents: final report of the American Institute of Nutrition ad hoc writing committee on the reformulation of the AIN-76A rodent diet.* J Nutr, 1993. **123**(11): p. 1939-51.
23. Lapre, J.A. and R. Van der Meer, *Diet-induced increase of colonic bile acids stimulates lytic activity of fecal water and proliferation of colonic cells.* Carcinogenesis, 1992. **13**(1): p. 41-4.
24. Burton, *K., A study of the conditions and mechanisms of the diphenylamine reaction for the colorimetric estimation of deoxyribonucleic acid*. Biochem J, 1956. **62**: p. 315-323.
25. Lapre, J.A., et al., *Lytic effects of mixed micelles of fatty acids and bile acids*. Am J Physiol, 1992. **263**(3 Pt 1): p. G333-7.
26. Bligh, E.G. and W.J. *Dyer, A rapid method of total lipid extraction and purification.* Canadian journal of biochemistry and physiology, 1959. **37**(8): p. 911-917.

## Claims

1. Use of a source of chlorophyll in the manufacture of a composition for prevention or reduction of cytotoxic effects of heme on the intestinal mucosa.

2. A use according to claim 1, whereby the prevention or reduction of cytotoxic effects of heme on the intestinal mucosa prevents or reduces the risk of developing an intestinal neoplasm, of which in particular colon-cancer.

3. A use according to claims 1 or 2, for prevention or reduction of cytotoxic effects of heme in a subject that is on a diet rich in heme.

4. A use according to any one of claims 1 - 3, wherein the amount of the amount of the source of chlorophyll that is administered or consumed provides for chlorophyll or analogues thereof in an amount that is 0.1 to 10 times the amount of dietary heme on a molar basis.

5. A use according to any one of claims 1 - 4, wherein the composition is a pharmaceutical or neutraceutical composition and wherein the prevention or reduction comprises the administration of the composition.

6. A use according to any one of claims 1 - 4, wherein the composition is a food product and wherein the prevention or reduction comprises the consumption of the food product.

7. A use according to claim 6, wherein the food product is enriched with a source of chlorophyll.

8. A use according to claims 6 or 7, wherein a source of chlorophyll comprised in the food product is an edible plant material comprising chlorophyll.

9. A use according to any one of claims 6 - 8, wherein the food product further comprises one or more ingredients comprising heme.

10. A use according to claim 9, wherein the food product is an instant meal.

11. A use according to any one of claims 1 - 10, wherein the source of chlorophyll is combined with a source of calcium in the manufacture of a composition for prevention or reduction of cytotoxic effects of heme on the intestinal mucosa.

12. A formulation comprising a source of chlorophyll and a source of calcium, whereby the formulation comprises dosage units for the source of chlorophyll of 0.2 - 2.0 mmol of chlorophyll per kg dry weight of diet or an analogue thereof and dosage units for the source of calcium of 1000 - 5000 mg calcium per kg of dry weight of diet.

13. A formulation according to claim 12, whereby the formulation comprises separate dosage units for the source of chlorophyll component and the source of calcium.

## Amended claims

### Amended claims in accordance with Rule 86(2) EPC.

**1.** A method for the prevention or reduction of cytoxic effects of heme on the intestinal mucosa, the method comprising the administration or consumption of a composition comprising a source of chlorophyll.

**2.** A method according to claim 1, whereby the method is a method for prevention or reduction of the risk of developing an intestinal neoplasm, of which in particular colon-cancer.

**3.** A method according to claims 1 or 2, whereby the method is a method for prevention or reduction of cytotoxic effects of heme in a subject that is on a diet rich in heme.

**4.** A method according to any one of claims 1 - 3, wherein the amount of the amount of the source of chlorophyll that is administered or consumed provides for chlorophyll or analogues thereof in an amount that is 0.1 to 10 times the amount of dietary heme on a molar basis.

**5.** A method according to any one of claims 1 - 4, wherein the composition is a pharmaceutical or neutraceutical composition and wherein the method comprises the administration of the composition.

**6.** A method according to any one of claims 1 - 4, wherein the composition is a food product and wherein the method comprises the consumption of the food product.

**7.** A method according to claim 6, wherein the food product is enriched with a source of chlorophyll.

**8.** A method according to claims 6 or 7, wherein a source of chlorophyll comprised in the food product is an edible plant material comprising chlorophyll.

**9.** A method according to any one of claims 6 - 8, wherein the food product further comprises one or more ingredients comprising heme.

**10.** A method according to claim 9, wherein the food product is an instant meal.

**11.** A method according to any one of claims 1 - 10, wherein the source of chlorophyll is combined with a source of calcium in the manufacture of a composition for prevention or reduction of cytotoxic effects of heme on the intestinal mucosa.

**12.** A formulation comprising a source of chlorophyll and a source of calcium, whereby the formulation comprises dosage units for the source of chlorophyll of 0.2 - 2.0 mmol of chlorophyll per kg dry weight of diet or an analogue thereof and dosage units for the source of calcium of 1000 - 5000 mg calcium per kg dry weight of diet.

**13.** A formulation according to claim 12, whereby the formulation comprises separate dosage units for the source of chlorophyll component and the source of calcium.
